# EUROPEAN PATENT APPLICATION

(11) **EP 2 108 316 A1**
(43) Date of publication of application: **14.10.2009**
(21) Application number: 09251049.4
(22) Date of filing: 07.04.2009
(51) Int. Cl.: A61B 17/04, A61B 17/00, A61B 17/06

(54) **Loop suture**

(30) Priority: 11.04.2008 US 44131 P; 23.03.2009 US 409094
(71) Applicant: Tyco Healthcare Group LP, North Haven, CT 06473 (US)
(72) Inventor: Cohen, Matthew D., Berlin, CT 06037 (US)
(74) Representative: Soames, Candida Jane

(57) **Abstract**

The present disclosure relates to a suture having a proximal end defining a first outer diameter and a distal end configured to accommodate the proximal end of the suture to form a closed loop stitch.

## Description

### CROSS-REFERENCE TO RELATED APPLICATION

The present application claims the benefit of and priority to U.S. Provisional Application Ser. No. 61/044,131, filed on April 11, 2008, the entire disclosure of which is incorporated herein by reference.

### BACKGROUND

### Technical Field

The present disclosure relates to sutures and more particularly to sutures which include a proximal end having a first outer diameter and a distal end having an inner hollow region with an inner diameter which is configured to accommodate at least a portion of the first outer diameter of the proximal end of the suture to form a stitch.

### Background of Related Art

Sutures are well known for tying approximated tissues of a wound together at intervals along the length of the wound. Although sutures have been proven to sufficiently close a wound, the process can be somewhat time consuming depending upon the handling characteristics of the suture and the wound location. For example, some suture materials are not easily tied into knots, or can not withstanding the constant movement through tissue when closing a larger wound and as a result break in the middle of the suturing process.

There are great advantages in providing a suture capable of closing a wound in tissue following surgery, i.e., plastic, general, or laparoscopic, which eliminates the need for tying a knot and also forms individual stitches thereby exposing the suture to less stress and decreasing the likelihood of the suture breaking before the wound is closed.

### SUMMARY

Accordingly, the present disclosure describes sutures which include an elongate body that has a promixal end and a distal end. The proximal end has a first outer diameter and the distal end has an inner hollow region with an inner diameter which is configured to receive at least a portion of the first outer diameter of the proximal end of the suture.

In embodiments, the sutures have an elongate body that has a distal end and a proximal end. The proximal end has first outer diameter, and the distal end has second outer diameter which may be greater than the first outer diameter of the proximal end. The distal end may further include an inner hollow region defining an inner diameter within the distal end which is configured to receive at least a portion of the first outer diameter of the proximal end. Methods of suturing tissue are also described.

The present disclosure also describes a suture as herein described for use in a method of suturing tissue wherein a method comprises the steps of passing the proximal end of the suture through the tissue; and engaging the distal end of the suture with at least a portion of the proximal end of the suture and forming a closed loop.

### BRIEF DESCRIPTION OF THE DRAWINGS

Various embodiments are illustrated and described herein, wherein:
FIG. 1A is a side view of a suture containing a distal end having an inner hollows region defined therein in accordance with one embodiment of this disclosure;
FIG. 1B is a side view of a suture containing a tip in accordance with one embodiment of this disclosure;
FIG. 2A is a side view of a suture containing at least one slot in accordance with one embodiment of this disclosure;
FIG. 2B is a side view of a suture forming a closed loop stitch in accordance with one embodiment of this disclosure;
FIG. 2C is a side view of a suture containing a distal end having an inner hollow region defined therein including internal friction-enhancing members.
FIG. 3 is a side view of suture containing a plug in accordance with one embodiment of this disclosure; and
FIGS. 4A-4C are side views of a suture containing a distal end having an inner hollow region defined therein including friction-enhancing members.

### DETAILED DESCRIPTION OF THE EMBODIMENTS

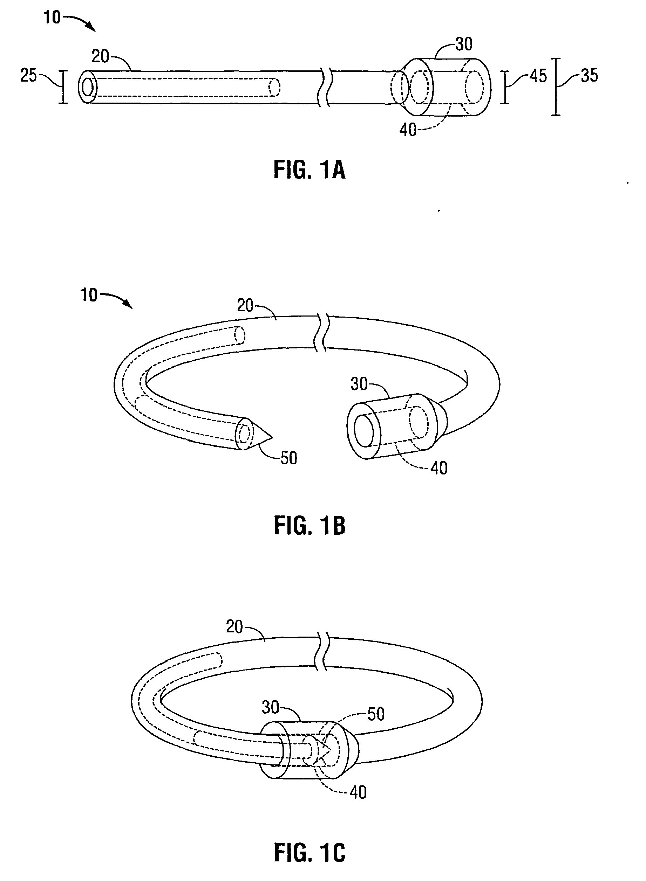

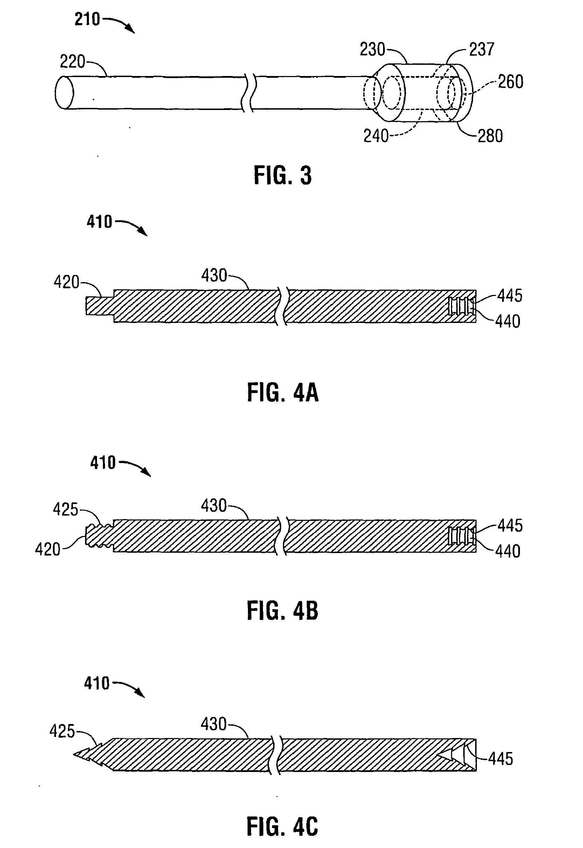

Turning now to FIG. 1 A, suture 10 is shown as a hollow monofilament having an elongate body which includes a proximal end 20 defining a first outer diameter 25 and a distal end 30 defining a second outer diameter 35 which is greater than or equal to first outer diameter 25. Distal end 30 also includes hollow region 40 defining an inner diameter 45 of distal end 30 which is configured to receive at least a portion of first outer diameter 25 of proximal end 20 of suture 10 to form a closed loop stitch. It is envisioned that suture 10 may be a monofilament or a multifilament suture (see FIGS. 4A-C) or a combination thereof and proximal end 20 may be hollow or solid or any combination thereof.

Suture 10 may be made from any material suitable for manufacturing surgical sutures or ligatures. Suture materials include for example any bioabsorbable, non-bioabsorbable, synthetic or natural materials and combinations thereof. Some suitable examples of absorbable materials include trimethylene carbonate, caprolactone, dioxanone, glycolic acid, lactic acid, glycolide, lactide, homopolymers thereof, copolymers thereof, and combinations thereof. Some specific examples of suitable non-absorbable materials which may be utilized to form the suture include polyolefins, such as polyethylene, polypropylene, copolymers of polyethylene and polypropylene, and blends of polyethylene and polypropylene. Some other useful materials include nylons, and cat-gut.

It is envisioned that the suture described herein may also be made from biomaterials not commonly associated as suture materials. Since the sutures are capable of forming a closed stitch loop which does not require the formation of a knot, these sutures can be formed of biomaterials which do not possess a minimal ability to form knots. One example of such a material would include biomaterials which having a low coefficient of friction. Another example would include biomaterials displaying a very high modulus.

As shown in FIG. 1A, in some embodiments, hollow region 40 is sealed within distal end 30 of suture 10 and is capable of storing at least one bioactive agent. In these embodiments, hollow region 40 can also serve as a vehicle for delivery of the bioactive agent. It is envisioned that at least a portion of proximal end 20 of suture 10 will penetrate into distal end 30 and enter into inner hollow region 40 to interact with and possibly release a bioactive agent stored therein. In embodiments where the bioactive agent is an adhesive, proximal end 20 may be positioned more permanently within inner hollow region 40 of distal end 30 after reacting with the adhesive.

The term "bioactive agent", as used herein, is used in its broadest sense and includes any substance or mixture of substances that have clinical use. Consequently, bioactive agents may or may not have pharmacological activity per se, e.g., a dye, or fragrance. Alternatively a bioactive agent could be any agent which provides a therapeutic or prophylactic effect, a compound that affects or participates in tissue growth, cell growth, cell differentiation, an anti-adhesive compound, a compound that may be able to invoke a biological action such as an immune response, or could play any other role in one or more biological processes. It is envisioned that the bioactive agent may be positioned on any part of the sutures described herein. For example, the bioactive agent may be simply coated on an outer or inner surface of the suture or combined with the material used to form the suture or may impregnate the suture surface. In addition, the inner hollow region may act as a reservoir in storing the bioactive agent. The bioactive agent may be positioned on the suture in any amount, configuration, and suitable form of matter, i.e., films, powders, liquids, gels and the like.

Examples of classes of bioactive agents which may be utilized in accordance with the present disclosure include antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, anti-adhesives, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, and enzymes. It is also intended that combinations of bioactive agents may be used.

Suitable antimicrobial agents which may be included as a bioactive agent stored within the suture described herein includes triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a bioactive agent in the present disclosure.

Other bioactive agents which may be included as a bioactive agent within the sutures described herein include: lubricants; local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g. oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anti-cancer agents; anti-convulsants; anti-emetics; antihistamines; antiinflammatory agents such as hormonal agents, hydrocortisone, prednisolone, prednisone, non-hormonal agents, allopurinol, indomethacin, phenylbutazone and the like; prostaglandins and cytotoxic drugs; estrogens; antibacterials; antibiotics; anti-fungals; anti-virals; anticoagulants; anticonvulsants; antidepressants; antihistamines; and immunological agents.

Other examples of suitable bioactive agents which may be included within the suture described herein include viruses and cells, peptides, polypeptides and proteins, analogs, muteins, and active fragments thereof, such as immunoglobulins, antibodies, cytokines (e.g. lymphokines, monokines, chemokines), blood clotting factors, hemopoietic factors, interleukins (IL-2, IL-3, IL-4, IL-6), interferons (β-IFN, (a-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, gonadotropins (e.g., FSH, LH, CG, etc.), hormones and hormone analogs (e.g., growth hormone), vaccines (e.g., tumoral, bacterial and viral antigens); somatostatin; antigens; blood coagulation factors; growth factors (e.g., nerve growth factor, insulin-like growth factor); protein inhibitors, protein antagonists, and protein agonists; nucleic acids, such as antisense molecules, DNA and RNA; oligonucleotides; polynucleotides; and ribozymes.

Turning now to FIG. 1B, suture 10 is shown to include to a tip 50 capable of penetrating tissue. In addition, where distal end 30 is closed, tip 50 can serve to penetrate distal end 30 to obtain access to hollow region 40 and form a closed loop stitch, as shown in FIG. 1C. In embodiments, tip 50 may be formed from the suture material. In embodiments, tip 50 may be a surgical needle connected to the suture material. It is envisioned that suture 10 can be connected to any suitable surgical needle capable of penetrating tissue, closing a wound and entering into hollow region 40 of distal end 30 of suture 10. Some non-limiting examples include curved needles, straight needles, tapered needles, triangular-body needles, round body needles, blunt and trocar needles. It is further envisioned that the sutures described herein may be attached to a suture needle using any conventional method known to those skilled in the art including swaging, crimping, heat-shrinking, adhesives, male/female mating engagement and so forth.

In some embodiments, suture 10 may include a tip 50 which is designed and configured to penetrate through tissue, approximate tissue and/or close a wound. Tip 50 may be blunt, sharp or any combination thereof and can be made from any suitable material capable of penetrating tissue to close a wound. Some useful examples of suitable materials include, but are not limited too, metals, such as stainless steels, metal alloys, shape memory alloys, such as Nitinol, and any shapeable polymeric materials, such as lactide, glycolide, caprolactone, and the like.

As shown in FIG. 1 B, tip 50 may be preformed and positioned within proximal end 20 of suture 10. In some embodiments, an adhesive, such as a cyanoacrylate, may be used to position tip 50 within proximal end 20 of suture 10. In still other embodiments, tip 50 may be heat-shrunk into position within suture 10.

In embodiments where tip 50 is made from a shape-memory alloy, it is envisioned that tip 50 may penetrate hollow region 40 of distal end 30 to form a close-loop stitch and then be exposed to an electrical, magnetic or temperature force which makes the shape-memory alloy return to its original shape and dimension which further tightens the closed-loop stitch creating a tighter and more secure wound closure.

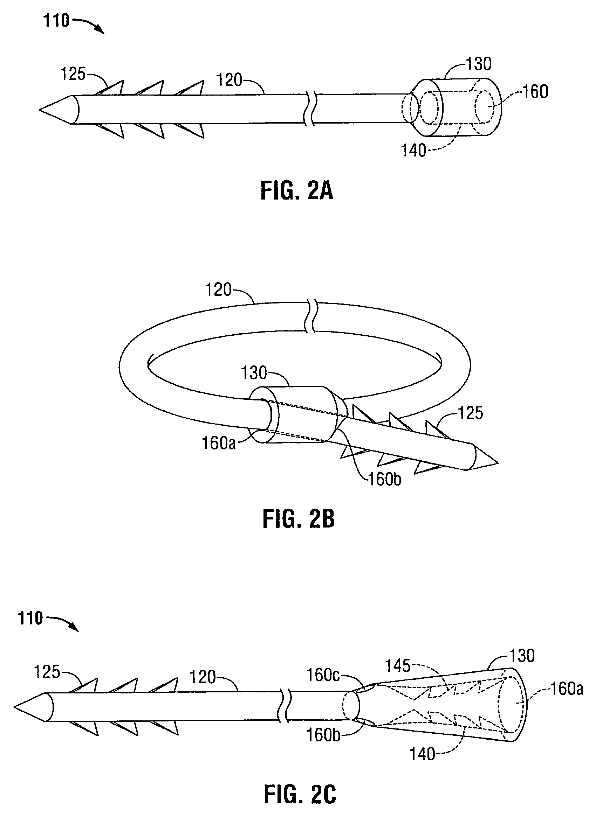

Turning now to the embodiment of FIG. 2A, flared distal end 130 of suture 110 is shown to further include slot 160. Slot 160 is positioned along flared distal end 130 to allow the passage of proximal end 120 of suture 110 into inner hollow region 140 to form a closed stitch loop. It is envisioned that slot 160 may be positioned on any portion of flared distal end 130 to allow entry of proximal end 120 into inner hollow region 140.

As shown in the embodiments of FIG. 2B, flared distal end 130 of suture 110 may include more than one slot 160a and 160b to allow proximal end 120 of suture 110 to penetrate at least a portion of flared distal end 130 and pass through inner hollow region 140 before exiting distal flared end 130 through additional slot 160b. Suture 110 is shown forming a 360º closed loop stitch. Such a stitch is designed to be formed quickly and does not necessarily require the use of a knot to keep the wound closed. Proximal end 120 is shown to include at least one external friction-enhancing member 125 which is designed to assist in holding suture 110 in the closed loop position. As shown, external friction-enhancing members 125 are barbs or barb-like structures positioned along the outer surface of proximal end 20 of suture 10. It is envisioned that the external friction-enhancing members may take any shape or geometric contour suitable for maintaining the proximal end of the suture within the inner hollow region. Suitable non-limiting examples include treads, bumps, grooves, spikes, ridges, and the like.

In FIG. 2C, inner hollow region 140 of flared distal end 130 of suture 110 is shown further including internal friction-enhancing members 145. Internal friction-enhancing members 145 may act as a ratchet type mechanism to secure the proximal end of the suture in the inner hollow region 130 to form a closed loop stitch. As shown, internal friction-enhancing members 145 are barbs or barb-like structures positioned along the inner surface of inner hollow region 130. It is envisioned that the internal friction-enhancing members may take any shape or geometric contour suitable for maintaining the proximal end of the suture within the inner hollow region. Suitable non-limiting examples include treads, bumps, grooves, spikes, ridges, and the like.

Turning now to FIG. 3, suture 210 is shown to include a plug 280 which is dimensioned to fit within slot 260. Since slot 260 creates an opening which interconnects inner hollow region 240 with an outer surface 237 of distal flared end 230, inner hollow region 240 may be unable to store a bioactive agent. Thus, plug 280 is designed to store at least one of the bioactive agents previously described herein and release the bioactive agent when penetrated by proximal end 220 of suture 210, which in some embodiments may further include a sharpened tip, upon entering or exiting inner hollow region 240 of distal flared end 230 of suture 210. Plug 280 may be made from any bioabsorbable or non-bioabsorbable material.

In the embodiments shown in FIGS. 4A-4C, the sutures are shown including at least one external friction-enhancing member and/or at least one internal friction-enhancing member. As shown, the external members may be added to the outer surface of the proximal end of the suture, and the internal members mat be positioned on the inner surface of the inner hollow region of the distal end of the suture, the inner surface of a slot, or any combination thereof. In FIG. 4A, a multifilament suture 410 is shown having an inner hollow region 440 which includes a plurality of internal friction-enhancing members 445 to assist in holding suture 410 in a closed loop position after passing through at least a portion of distal end 430. As further shown in Figs. 4B and 4C, external friction-enhancing members 425 and internal friction-enhancing members 445 may take any shape, dimension or contour capable of assisting with holding the suture in a closed loop stitch. Suitable friction-enhancing members may also be formed from any material capable of assisting in positioning at least a portion of the proximal end of the suture within the inner hollow region of the distal end of the suture.

It is envisioned that the sutures described herein may be made of any suitable size, shape and dimension to close a wound in living tissue. It is further envisioned that the sutures described herein may be used to close a wound in any type of tissue. In particularly useful embodiments, the sutures may be used to close sub-dermal tissue, such as wounds common to plastic, laparoscopic and general surgeries.

In embodiments, the sutures described herein may be used to suture wounded tissues and form knotless wound closures. Methods of suturing tissue include the steps of: providing a suture having a proximal end with a first outer diameter and a distal end having an inner hollow region with an inner diameter which is configured to receive at least a portion of the first outer diameter of the proximal end of the suture, passing the proximal end of the suture through the tissue; and engaging the distal end of the suture with at least a portion of the proximal end of the suture and forming a closed loop. In embodiments, the closed loop can vary in size and is adjustable to apply the appropriate amount of tension and force to keep the wound closed.

The sutures described herein may be made using any known method for forming a suture. Some non-limiting examples include, molding, extruding, coextruding, and the like. In particular embodiments, the sutures described herein may begin as a hollow monofilament made of a suitable suture material. The distal end the hallow suture is then expanded to allow the proximal end of the suture to be received within the inner hollow region of the expanded distal end of the suture. The distal end can be expanded using any suitable method, including but not limited to, the use of the heat, pressure, physical force and any combination thereof. In embodiments, a preformed tip may be positioned in the proximal end of the hollow suture using any suitable means known to those skilled in the art.

Also contemplated herein is a suture according to the invention for use in a method of suturing tissue wherein the method comprises the steps of passing the proximal end of the suture through the tissue; and engaging the distal end of the suture with at least a portion of the proximal end of the suture and forming a closed loop.

It will be understood that various modifications may be made to the embodiments disclosed herein. Therefore, the above description should be construed as limiting, but merely as an exemplification of preferred embodiments. Those skilled in the art will envision other modifications within the scope and spirit of the present disclosure. Various modifications and variations of the suture and uses thereof will be apparent to those skilled in the art from the foregoing detailed description. Such modifications and variations are intended to come within the scope of the following claims.

## Claims

1. A suture comprising:
an elongate body having a proximal end and a distal end,
the proximal end having a first outer diameter, and
the distal end having an inner hollow region with an inner diameter which is configured to receive at least a portion of the first outer diameter of the proximal end of the suture.

2. The suture of claim 1 wherein the suture comprises a monofilament; or
the suture of claim 1 wherein the suture comprises a multifilament; or
the suture of claim 1 wherein the suture comprises a bioabsorbable material; or
the suture of claim 1 wherein the suture comprises a non-bioabsorbable material; or
the suture of claim 1 wherein the suture forms a closed loop; or
the suture of claim 1 wherein the distal end of the suture is flared; or
the suture of claim 1 wherein the distal end of the suture has a second outer diameter that is greater than the first outer diameter of the proximal end of the suture.

3. The suture of claim 1 further comprising at least one slot on the distal end of the suture to allow at least a portion of a the proximal end of the suture to be received by the distal end of the suture.

4. The suture of claim 3 further comprising a plug engaged to at least one of the slots of the flared distal end, wherein the plug is dimensioned to frictionally fit within the slot of the distal end of the suture.

5. The suture of claim 1 wherein the proximal end of the suture is hollow; or
the suture of claim 1 wherein the suture is hollow; or
the suture of claim 1 wherein the proximal end of the suture further comprises a pointed tip for penetrating tissue.

6. The suture of claim 5 wherein the pointed tip is a surgical needle.

7. The suture of claim 6 wherein the pointed tip is made from a material selected from the group consisting of stainless steel, shape memory alloys, metal alloys and combinations thereof.

8. The suture of claim 1 wherein the suture further comprises at least one external friction-enhancing member.

9. The suture of claim 8 wherein the external friction-enhancing member is selected from the group consisting of barbs, grooves, treads, bumps, ridges, adhesives and combinations thereof.

10. The suture of claim 9 wherein the external friction-enhancing member is positioned on the proximal end of the suture.

11. The suture of claim 1 wherein the suture further comprises at least one internal friction-enhancing member.

12. The suture of claim 11 wherein the internal friction-enhancing member is selected from the group consisting of barbs, grooves, treads, bumps, ridges, adhesives and combinations thereof.

13. The suture of claim 12 wherein the internal friction-enhancing member is positioned in the inner hallow region of the distal end of the suture.

14. A suture comprising:
an elongate body having a proximal end and a distal end,
the proximal end having a first outer diameter, and
the distal end having a second outer diameter and an inner hallow region defining an inner diameter within the distal end, wherein the distal end is configured to receive at least a portion of the first outer diameter of the proximal end.

15. A suture having a proximal end with a first outer diameter and a distal end having an inner hollow region with an inner diameter which is configured to receive at least a portion of the first outer diameter of the proximal end of the suture for use in a method of suturing tissue wherein the method comprises the steps of passing the proximal end of the suture through the tissue; and engaging the distal end of the suture with at least a portion of the proximal end of the suture and forming a closed loop.
